Europäisches Patentamt

European Patent Office

Office européen des brevets

(11) Veröffentlichungsnummer: **0 014 449**
**B1**

(12) **EUROPÄISCHE PATENTSCHRIFT**

(45) Veröffentlichungstag der Patentschrift:
**27.04.83**

(51) Int. Cl.³: **C 07 D 235/26**

(21) Anmeldenummer: **80100536.4**

(22) Anmeldetag: **04.02.80**

(54) Verfahren zur Herstellung von 5-Nitrobenzimidazolon-(2).

(30) Priorität: **10.02.79 DE 2905126**

(43) Veröffentlichungstag der Anmeldung:
**20.08.80 Patentblatt 80/17**

(45) Bekanntmachung des Hinweises auf die Patenterteilung:
**27.04.83 Patentblatt 83/17**

(84) Benannte Vertragsstaaten:
**BE CH DE FR GB IT**

(56) Entgegenhaltungen:
**,,Monatshefte für Chemie", Bd. 107, Heft 4, Juli/ August 1976, S. 1307-1310, Wien, AT. H. Schindlbauer** et al.: **,,Zur direkten Nitrierung des Benzimidazolons und der Reduktion einiger dieser Nitrierungsprodukte".**

**,,Chemical Abstract", Bd. 51, Nr. 17, 10. September 1957, Spalte 12882f, L.S. Efros** et al.: **,,Imidazole derivatives. XV. Nitration of benzimidazolone and 1,3-dimethylbenzimidazolone".**

(73) Patentinhaber: **HOECHST AKTIENGESELLSCHAFT, Postfach 80 03 20, D-6230 Frankfurt/Main 80 (DE)**

(72) Erfinder: **Mees, Bernhard, Dr., Gräfliche Strasse 31, D-6239 Eppstein/Taunus (DE)**
Erfinder: **Ribka, Joachim, Dr., Rügener Strasse 4, D-6050 Offenbach/Main (DE)**

## Verfahren zur Herstellung von 5-Nitrobenzimidazolon-(2)

5-Nitribenzimidazolon-(2) dient als Ausgangsmaterial zur Herstellung der entsprechenden Aminoverbindung, die als Diazokomponente sowie in Form der entsprechenden N-Acetoacetylverbindung als Kupplungskomponente für Azofarbmittel, insbesondere Pigmente, verwendet wird.

Die Nitrierung von Benzimidazolon-(2) ist bereits bekannt. So ist in „Zhur. Obshchei Khim.", 27 (1957), S. 127-135, beschrieben, dass man Benzimidazolon in konzentrierter Schwefelsäure auflösen und unter heftigem Rühren mit einer Mischung aus konzentrierter Schwefelsäure und der etwa äquimolaren Menge an rauchender Salpetersäure bei 0°C umsetzen kann, wobei nach einer umständlichen Aufarbeitung 5-Nitrobenzimidazolon-(2) in einer Ausbeute von 68,5% isoliert wird. Unter den gleichen Reaktionsbedingungen entsteht mit der etwa dreifach molaren Menge an rauchender Salpetersäure jedoch 5,6-Dinitrobenzimidazolon-(2) in einer Rohausbeute von 94%. Abgesehen von der aufwendigen Aufarbeitung ist bei diesem Verfahren somit von Nachteil, dass neben der erwünschten Mononitroverbindung auch höhere Nitroverbindungen schon bei tiefen Temperaturen erhalten werden.

Aus „Monatshefte für Chemie", 107 (1976), S. 1307-1310, ist es bekannt, Benzimidazolon in einer Mischung aus Acetanhydrid und Eisessig mit einer Nitriersuspension aus der etwa 4,5fach molaren Menge an konzentrierter Salpetersäure, Eisessig und Harnstoff bei einer Temperatur bis zu 30°C umzusetzen. Hierbei wird 5-Nitrobenzimidazolon-(2) zwar in einer Ausbeute von 90,5% erhalten; unbefriedigend ist jedoch der Einsatz des organischen Lösemittelgemisches, der hohe Überschuss an konzentrierter Salpetersäure und die damit verbundene aufwendige Aufarbeitung der anfallenden verdünnten Säuren. Wird bei 40°C nur mit konzentrierter Salpetersäure nitriert, dann wird die 5-Nitro- und die 5,6-Dinitroverbindung im Verhältnis von etwa 1:5 erhalten; bei 70°C wird nur noch die Dinitroverbindung isoliert.

Nach diesen bekannten Verfahren wurde also bei der Nitrierung von Benzimidazolon-(2) ein wasserbindendes Reaktionsmedium eingesetzt, nämlich entweder konzentrierte Schwefelsäure oder Acetanhydrid. Überraschenderweise wurde nun gefunden, dass die Nitrierungsreaktion sehr glatt im Reaktionsmedium Wasser verläuft.

Gegenstand der Erfindung ist deshalb ein Verfahren zur Herstellung von 5-Nitrobenzimidazolon-(2) durch Umsetzung von Benzimidazolon-(2) mit Salpetersäure in einem flüssigen Medium, das dadurch gekennzeichnet ist, dass das flüssige Medium Wasser ist und die Nitrierung bei einer Temperatur von 20-100°C erfolgt.

Im folgenden werden bevorzugte Ausgestaltungen der Erfindung näher beschrieben, wobei Prozentangaben sich auf das Gewicht beziehen:

Die Konzentration der eingesetzten wässerigen Salpetersäure soll zweckmässig 10% nicht unterschreiten, da sonst selbst bei Temperaturen in der Nähe von 100°C die Reaktion zu langsam verläuft. Arbeitet man bei einer Temperatur von etwa 80-85°C, so genügt eine Konzentration von 10-12%, um ein vernünftiges Zeit/Umsatz-Verhältnis zu erhalten.

Arbeitet man an der Untergrenze des beanspruchten Temperaturbereiches, so ist eine Konzentration der Salpetersäure von mindestens 45% erforderlich. Höhere Konzentrationen der Salpetersäure sind jedoch unzweckmässig, da hierbei die Bildung von höher nitrierten Produkten erfolgen kann. Ausserdem steigt die Löslichkeit des Nitrobenzimidazolons im Reaktionsmedium, so dass nach Beendigung der Reaktion relativ viel Wasser zur vollständigen Ausfällung der Nitroverbindung zugesetzt werden muss.

Aus den genannten Gründen ist somit ein Konzentrationsbereich zwischen etwa 10 und 45% bevorzugt, wobei die Reaktionstemperatur zwischen 30 und 80°C liegt.

Das Molverhältnis von Salpetersäure zu Benzimidazolon lässt sich in relativ weiten Grenzen variieren. Da die Konzentration der Salpetersäure gegen Ende der Umsetzung 10% nicht unterschreiten soll, damit die Nitrierung noch vollständig verläuft, wählt man das Molverhältnis zweckmässig so, dass es zwischen 2 und 4 mol Salpetersäure pro Mol Benzimidazolon liegt.

Bei einer zweckmässigen Ausgestaltung des erfindungsgemässen Verfahrens wird das Benzimidazolon unter Rühren in eine ca. 26%ige Salpetersäure eingetragen. Die Nitrierung setzt bei etwa 30°C ein und wird bei etwa 35°C zu Ende geführt.

Ein besonderer Vorteil des erfindungsgemässen Verfahrens liegt darin, dass infolge der einheitlich verlaufenden Nitrierung die Mutterlauge wieder auf die Ausgangskonzentration aufgestärkt und neu eingesetzt werden kann. Durch diese Rückgewinnung der Salpetersäure ist es also nur erforderlich, die minimalen Verluste auszugleichen, die durch die beim Isolieren anhaftende Mutterlauge entstehen. In der Bilanz ist somit praktisch nur 1 mol Salpetersäure pro Mol Benzimidazolon erforderlich. Damit entstehen auch keine Aufarbeitungsprobleme hinsichtlich der wässerigen Salpetersäure, insbesondere keine Umweltbelastung.

In den folgenden Beispielen wird die Erfindung näher erläutert. Volumen- und Gewichtsteile stehen hierbei im Verhältnis von Kilogramm zu Liter; die Prozentangaben beziehen sich auch hier auf das Gewicht.

*Beispiel 1:*

In einem Rührgefäss werden 600 Volumenteile Wasser vorgelegt und bei Raumtemperatur unter Rühren und Kühlen 150 Volumenteile technischer Salpetersäure (99%ig, d = 1,51) zugegeben, so dass nun 750 Volumenteile einer 26%igen Salpetersäure vorliegen (d = 1,157). Bei 25°C trägt man 180 Gewichtsteile Benzimidazolon in Form eines ca. 75%igen wässerigen Presskuchens (entsprechend 134 Gewichtsteile = 1 mol) ein. Die Temperatur soll dabei nicht über 35°C steigen. Die anfangs klare Lösung wird bald trüb und das 5-Nitrobenzimidazolon beginnt auszufallen. Nach Beendigung des Eintragens lässt man noch bei 35°C 1 h nachrühren. Dann wird mit 1000 Volumenteilen Wasser verdünnt und ½ h weitergerührt. Anschliessend wird über eine säurefeste Nutsche abgesaugt, mit Wasser fast neutral gewaschen und die Nitroverbindung bei 105°C getrocknet.

Man erhält 173 Gewichtsteile 5-Nitrobenzimidazolon-(2), entsprechend 97% Ausbeute, bezogen auf eingesetztes Benzimidazolon, welches dünnschichtchromatographisch einheitlich ist und einen Schmelzpunkt von 308°C zeigt.

*Beispiel 2:*

In einem Rührgefäss werden 840 Gewichtsteile Wasser vorgelegt und bei Raumtemperatur unter Rühren und Kühlen 161 Gewichtsteile einer 99%igen technischen Salpetersäure (d = 1,51) zugegeben. Man erhält 1001 Gewichtsteile einer 16%igen Salpetersäure. Die fertige Mischung wird auf 50°C erhitzt und unter Rühren 180 Gewichtsteile Benzimidazolon in Form eines ca. 75%igen wässerigen Presskuchens (1 mol) eingetragen. Die Reaktion wird so geführt, dass innerhalb von 2 h die Nitrierung bei 50-75°C abläuft. Man erhitzt noch ½ h auf 90°C, um die Filtrierbarkeit des 5-Nitrobenzimidazolons-(2) zu verbessern, kühlt dann auf Raumtemperatur ab und saugt über eine säurefeste Nutsche ab. Die weitere Aufarbeitung entspricht dem Beispiel 1.

Die Ausbeute an Nitroverbindung liegt bei 96%, bezogen auf eingesetztes Benzimidazolon.

*Beispiel 3:*

Die Mutterlauge (etwa 800 Gewichtsteile ca. 10%ige wässerige Salpetersäure) aus Beispiel 2 wird mit 65 Gewichtsteilen einer 99%igen Salpetersäure aufgestärkt und die Nitrierung gemäss Beispiel 2 durchgeführt.

Man erhält das 5-Nitrobenzimidazolon-(2) dünnschichtchromatographisch einheitlich in einer Ausbeute von 98%, bezogen auf eingesetztes Benzimidazolon.

**Patentansprüche**

1. Verfahren zur Herstellung von 5-Nitrobenzimidazolon-(2) durch Umsetzung von Benzimidazolon-(2) mit Salpetersäure in einem flüssigen Medium, dadurch gekennzeichnet, dass das flüssige Medium Wasser ist und die Nitrierung bei einer Temperatur zwischen 20 und 100°C erfolgt.

2. Verfahren nach Anspruch 1, dadurch gekennzeichnet, dass die Reaktionstemperatur zwischen 30 und 80°C liegt.

3. Verfahren nach den Ansprüchen 1 und 2, dadurch gekennzeichnet, dass die Konzentration der Salpetersäure 10 bis 45 Gew.% beträgt.

4. Verfahren nach den Ansprüchen 1 bis 3, dadurch gekennzeichnet, dass pro Mol Benzimidazolon-(2) 2 bis 4 mol Salpetersäure eingesetzt werden.

5. Verfahren nach den Ansprüchen 1 bis 4, dadurch gekennzeichnet, dass die Mutterlauge eines vorhergehenden Ansatzes mit Salpetersäure auf die ursprüngliche Konzentration aufgestärkt wird.

**Claims**

1. A process for the preparation of 5-nitrobenzimidazolone-(2) by reacting benzimidazolone-(2) with nitric acid in a liquid medium, which comprises performing the nitration in water at a temperature of 20 to 100°C.

2. A process as claimed in claim 1, wherein the reaction temperature is 30 to 80°C.

3. A process as claimed in claims 1 and 2, wherein the concentration of the nitric acid is 10 to 45% by weight.

4. A process as claimed in claims 1 to 3, wherein 2 to 4 mol of nitric acid are added per mol of benzimidazolone-(2) to the reaction mixture.

5. A process as claimed in claims 1 to 4, wherein the mother liquor of a previous nitration is adjusted with concentrated nitric acid to the desired concentration and reacted with benzimidazolone-(2).

**Revendications**

1. Procédé pour préparer la nitro-5 benzimidazolone-2 par réaction de la benzimidazolone-2 avec l'acide nitrique dans un milieu liquide, caractérisé en ce que le milieu liquide est l'eau et en ce que la nitration est effectuée à une température comprise entre 20 et 100°C.

2. Procédé selon la revendication 1, caractérisé en ce que la température réactionnelle est comprise entre 30 et 80°C.

3. Procédé selon les revendications 1 et 2, caractérisé en ce que la concentration de l'acide nitrique est comprise entre 10 et 45% en poids.

4. Procédé selon l'une quelconque des revendications 1 à 3, caractérisé en ce qu'on met en jeu de 2 à 4 mol d'acide nitrique par mole de benzimidazolone-2.

5. Procédé selon l'une quelconque des revendications 1 à 4, caractérisé en ce que la liqueur mère provenant d'une opération antérieure est ramenée à la concentration de départ par addition d'acide nitrique.